# EUROPEAN PATENT SPECIFICATION

(11) **EP 1 547 550 B1**
(45) Date of publication and mention of the grant of the patent: **15.08.2007**
(21) Application number: 04030669.8
(22) Date of filing: 23.12.2004
(51) Int. Cl.: A61F 9/007

(54) **Vitreous body cutter and method for manufacturing same**
Schneidegerät für den Glaskörper und Verfahren zur Herstellung desselben
Moyen de coupe pour le corps vitré et méthode de fabriquer ledit moyen de coupe

(30) Priority: 25.12.2003 JP 2003429295
(43) Date of publication of application: 29.06.2005
(73) Proprietor: Nidek Co., Ltd., Gamagori-shi, Aichi (JP); Takashima Sangyo Corp., Suwa-shi, Nagano-ken (JP)
(72) Inventor: Ichikawa, Kazuo, 5695-6, Kanazawa Chino-shi Nagano-ken (JP); Ito, Tadahiko, 5695-6, Kanazawa Chino-shi Nagano-ken (JP); Osawa, Koji, Anjo-shi Aichi (JP)
(74) Representative: Schmitz, Hans-Werner

(56) References cited:
- EP-A- 0 514 057
- EP-A- 0 590 887
- US-A- 5 782 849
- US-A1- 2002 173 814

## Description

### BACKGROUND OF THE INVENTION

The present invention relates to a vitreous body cutter for cutting a vitreous body in an eye, vitreous body surgical equipment (apparatus) equipped with the vitreous body cutter, and a method for manufacturing the vitreous body cutter.

In a vitreous body cutter used in vitreous body surgery, a vitreous body of an eye is drawn by suction through a suction hole formed in a side surface in the vicinity of an extremity (a distal end) of a fixed, outer cylindrical (tubular) blade, to thus cause the vitreous body to fit into the suction hole. An inner cylindrical (tubular) blade is caused to axially reciprocate (move back and forth) with respect to the outer cylindrical blade (a guillotine type) or to rotate about its central axis (a rotary type), to thus excise the fitted vitreous body (see US Patent No. 6514268 (JP-A-2003-529402)).

Such a vitreous body cutter requires high-precision meshing engagement between the outer cylindrical blade and the inner cylindrical blade. However, the related-art vitreous body cutter is manufactured by machining the outer and inner cylindrical blades, which poses difficulty in ensuring

### SUMMARY OF THE INVENTION

A technical problem to be solved by the present invention is to provide a vitreous body cutter having an outer cylindrical cutter and an inner cylindrical cutter, between which high-precision meshing engagement is ensured; vitreous body surgical equipment having the vitreous body cutter; and a method for manufacturing the vitreous body cutter.

EP A 0 514 057 discloses a vitreous cutter for use in removing vitreous from the eye during intraocular surgery. The cutter includes -an outer tube having a side-facing opening with a perimeter edge forming a cutting blade. An inner tube is received coaxially in the outer tube and is provided at one end with a side facing opening whose perimeter edge forms a second cutting edge. The inner tube is rotatable about its axis relative to the outer tube to cause the side facing openings to alternately rotate into and out of alignment. This causes the cutting edges to sever vitreous sucked into the aligned openings by suction pressure applied to the opposite end of the inner tube.

EP-A 0 590 887 discloses a surgical instrument comprising inner and outer tubular members having a coating of tin-nickel alloy on the outer surface of the inner tubular member and/or the inner surface of the outer tubular member to provide an elongate bearing surface between the tubular members.

US-A 2002/0173814 refers to a method of operating a microsurgical instrument, such as a vitrectomy probe. The probe includes a port for receiving tissue and an inner cutting member. A flow of tissue is included into the port with a vacuum source.

This problem is solved by the vitreous body cutter of claim 1 and the corresponding manufacturing method of claim 6.

### BRIEF DESCRIPTION OF THE DRAWINGS

Fig. 1A is a schematic side cross-sectional view showing the shape of an extremity (a distal end) of an outer cylindrical (tubular) blade according to an embodiment and the shape of the vicinity thereof;
Fig. 1B is a schematic side cross-sectional view showing the shape of an extremity (a distal end) of an inner cylindrical (tubular) blade according to an embodiment and the shape of the vicinity thereof;
Fig. 2 is a schematic external view showing the inner cylindrical blade fitted into the outer cylindrical blade;
Figs. 3A and 3B are schematic side cross-sectional views showing the inner cylindrical blade fitted in the outer cylindrical blade;
Figs- 4A and 4B are schematic side cross-sectional views showing the inner cylindrical blade fitted in the outer cylindrical blade;
Figs. 5A, 5B, and 5C are schematic views showing a method for manufacturing the outer and inner cylindrical blades;
Fig. 6 is a schematic block diagram of vitreous body surgical equipment of the present embodiment; and
Fig. 7 is a schematic side cross-sectional view showing an embodiment in which a sensor is attached to a vitreous body cutter.

### DETAILED DESCRIPTION OF THE PREFERRED EMBODIMENTS

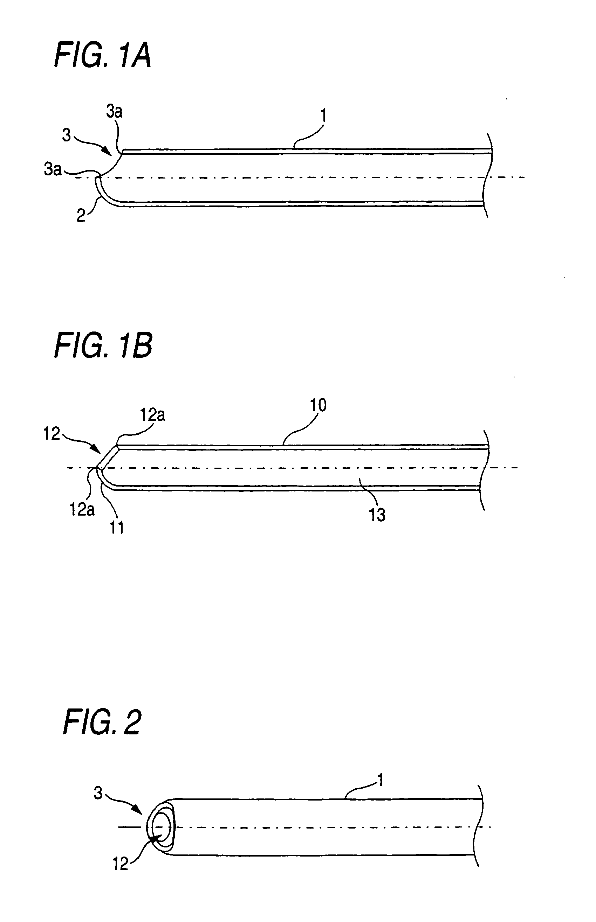

An embodiment according to the present invention will be described with reference the drawings. Fig. 1A is a schematic side cross-sectional view of an outer cylindrical (tubular) blade 1 . The outer cylindrical blade 1 is formed into a hollow cylindrical shape having an outer diameter of about 0.7 mm to about 1.5 mm and a thickness of about 0.05 mm to about 0.2 mm (therefore an inner diameter of about 0. 3 mm to about 1 - 4 mm) . A curved (rounded) surface 2 is formed at a distal end of the outer cylindrical blade 1. the reason for this is that consideration is taken for infliction of damage on a retina, which would otherwise be caused when the distal end of the outer cylindrical blade 1 comes into contact with the retina. As a matter of course, it may be the case that the curved surface 2 is not formed.

An opening 3 is formed in the distal end of the outer cylindrical blade 1 by cutting a portion of the curved surface 2. This opening 3 is a suction hole for drawing a vitreous body into the outer cylindrical blade 1 by suction, and an edge 3a on an interior-wall-side of the opening 3 acts as an outer blade. The shape of the interior wall of the outer cylindrical blade 1 essentially coincides with the shape of an external wall of an inner cylindrical (tubular) blade 10 to be described later.

Fig. 1B is a schematic side cross-sectional view of the inner cylindrical blade 10 housed in the outer cylindrical blade 1. The inner cylindrical blade 10 is fitted into the outer cylindrical blade 1 and retained (held) so as to be rotatable. The inner cylindrical blade 10 is formed into a hollow cylindrical shape whose outer diameter essentially coincides with the inner diameter of the outer cylindrical blade 1 (a clearance of micrometers to tens of micrometers exists between the inner wall of the outer cylindrical blade 1 and the external wall of the inner cylindrical blade 10).

A curved (round) surface 11 substantially coinciding with the inner wall of the curved surface 2 of the distal end of the outer cylindrical blade 1 is formed at the distal end of the inner cylindrical blade 10. An opening 12 is formed in the distal end of the inner cylindrical blade 10 by cutting a portion of the curved surface 11 . This opening 12 is a suction hole for drawing a vitreous body into the inner cylindrical blade 10 by suction, and an edge 12a on the external wall-side of the opening 12 acts as an inner blade.

A suction device and a rotary drive device are provided on a base-end-side of the inner cylindrical blade 10, and the inner cylindrical blade 10 is rotated about its central axis within the outer cylindrical blade 1. The vitreous body is drawn into a suction path 13 in the inner cylindrical blade 10 by suction through the opening 3 and the opening 12 (which will be described in detail later).

Fig. 2 is a schematic external view showing the inner cylindrical blade 10 fitted into the outer cylindrical blade 1. The vitreous body cutter of the present embodiment incises a vitreous body by rotating (rotationally moving) the inner cylindrical blade 10 with respect to the outer cylindrical blade 1 between a state where the opening 3 and the opening 12 overlap each other (an open state where the opening 12 can be seen through the opening 3) and a state where they do not overlap each other (a closed state where the opening 12 is not visible through the opening 3). When the opening 3 and the opening 12 overlap each other as shown in Fig. 2, the vitreous body is drawn into the suction path 13 in the inner cylindrical path 10 by suction.

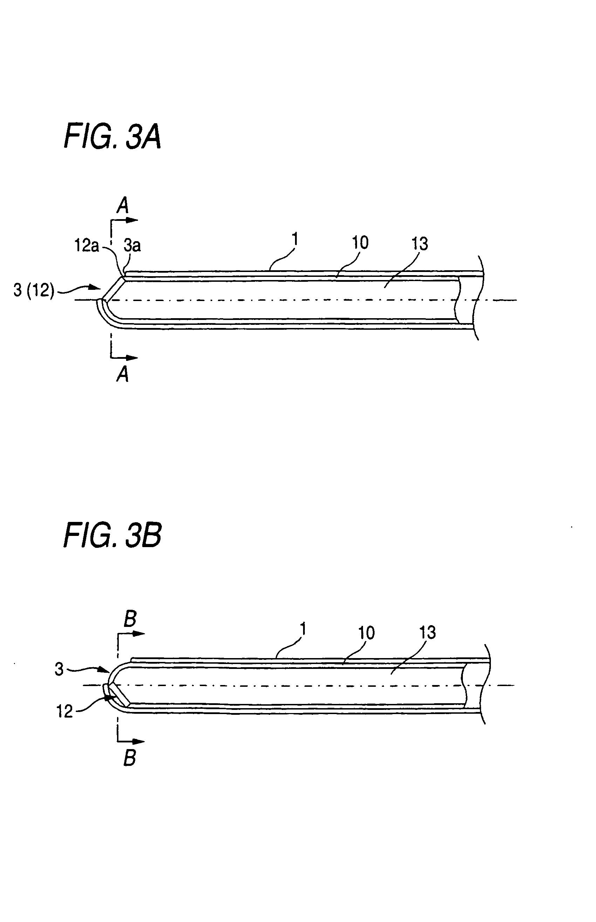

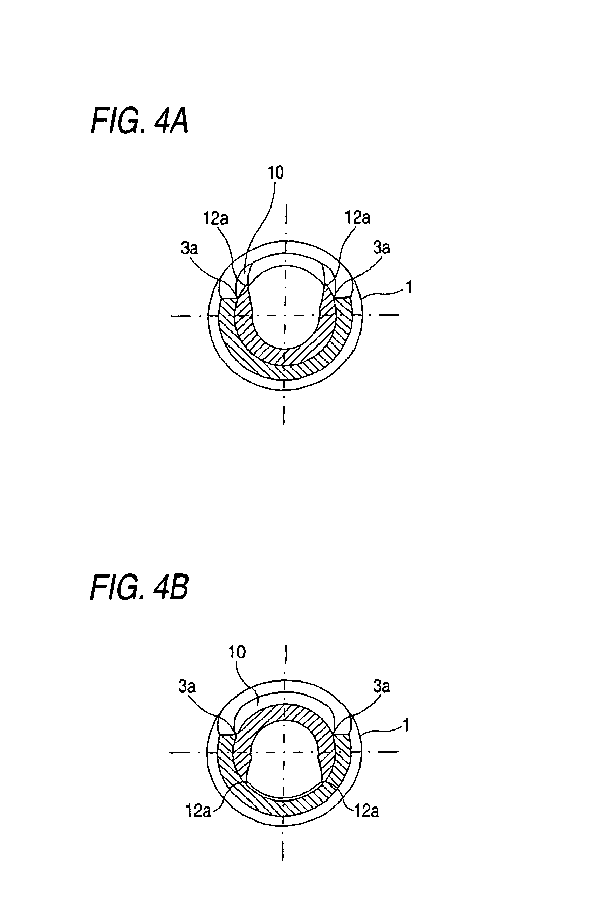

Fig. 3A is a schematic side cross-sectional view showing that the inner cylindrical blade 10 is fitted in the outer cylindrical blade 1 and that the opening 3 and the opening 12 overlap each other. Fig. 4A is a schematic side cross-sectional view along direction A shown in Fig. 3A. Fig. 3B is a schematic side cross-sectional view showing that that the opening 3 and the opening 12 do not overlap each other. Fig. 4A is a schematic side cross-sectional view along direction B shown in Fig. 3B-Asmentionedpreviously, when the opening 12 overlaps the opening 13, the vitreous body is drawn into the suction path 13 by suction through the openings 3 and 12. When the opening 12 does not overlap the opening 3 as a result of rotation of the inner cylindrical blade 10 within the outer cylindrical blade 1, the vitreous body is incised by meshing engagement between the edge 3a and the edge 12a.

The shape of the inner wall of the outer cylindrical blade 1 essentially coincides with the shape of the outer wall of the inner cylindrical blade 10. When the inner cylindrical blade 10 is fitted into the outer cylindrical blade 1, the blades can be fitted together without involvement of any substantial clearance between the blades (only a clearance sufficient for rotation exists). Consequently, the accuracy of meshing engagement between the edge 3a and the edge 12a is greatly enhanced, so that the vitreous body can be incised without excess labor.

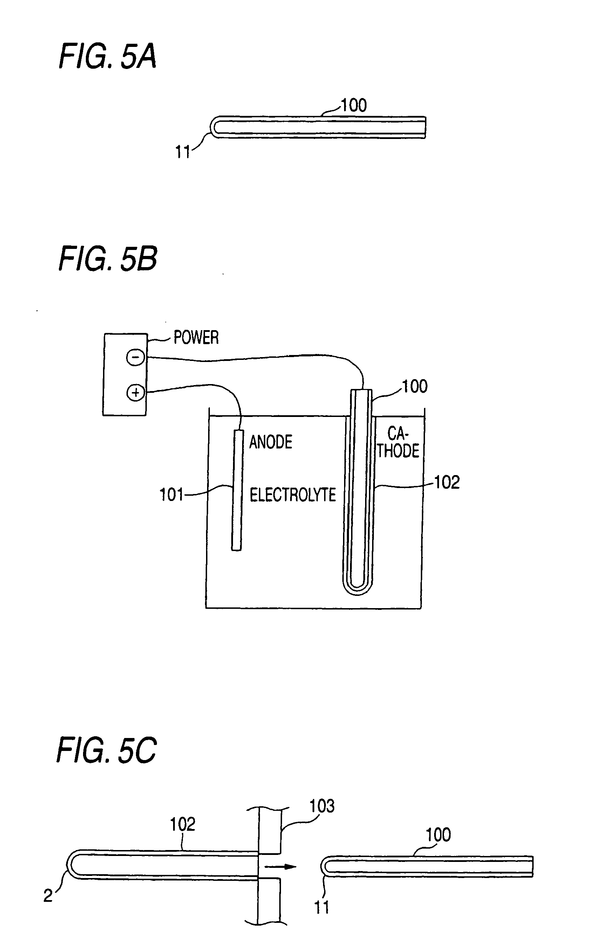

A method for manufacturing such an outer cylindrical blade 1 and such an inner cylindrical blade 10 will now be described with reference to Figs. 5A to 5C. First, the distal end of a pipe material which is of a size (has inner and outer diameters) suitable for use as the inner cylindrical blade 10 and is formed from metal (for example, stainless) is closed in advance by electrodeposition, welding or the like. Next, the distal end is formed into the curved surface 11 by cutting with a cutting tool or grinding with a grindstone. As a result, as shown in Fig. 5A, a metal matrix 100 which is to be formed into the inner cylindrical blade 10 is obtained.

As shown in Fig. 5B, the metal matrix 100 is placed as a cathode in an electrolyte for electroforming, which is made of nickel sulfamic acid solution, or the like, and a sulfur-containing nickel plate 101 is used as an anode. A predetermined voltage is applied across electrodes from a power source, to thus deposit nickel on the surface of the metal matrix 100. At this time, deposition of nickel is controlled by means of the voltage to be applied and an application time, so that nickel is deposited on the surface of the metal matrix 100 to a thickness required for the outer cylindrical blade 1 (a thickness of essentially 0. 05 mm to 0.2 mm in the embodiment). The metal matrix 100 is rotated within the electrolyte such that nickel is deposited to as uniform a thickness as possible over the overall metal matrix 100. Any rotational speed is allowable, so long as nickel is uniformly deposited over the entire metal matrix 100. For instance, the rotational speed is set so as to fall within a range of about 10 rpm to about 200 rpm.

When a nickel cylinder 102 formed from the metal matrix 100 with nickel deposited thereon is obtained by means of such an electro forming method, the metal matrix 100 having the nickel cylinder 102 is taken out of the electrolyte. Subsequently, as shown in Fig. 5C, the metal matrix 100 is released from the nickel cylinder 102. In this case, for instance, it is better to leave the nickel cylinder 102 in contact with a stopper 103, and to withdraw the metal matrix 100 away from the nickel cylinder 102. As a result, the shape of the outer wall of the metal matrix 100 which is to become the inner cylindrical blade 10 is transferred as the shape of the inner wall of the nickel cylinder 102 which is to become the outer cylindrical blade 1.

Next, the opening 3 is formed by cutting a portion of the distal end of the nickel cylinder 102 with an end mill or the like. At this time, the distal end of the end mill is not brought into contact with the curved surface 2 of the nickel cylinder 102, but is brought into contact at right angles to the nickel cylinder 102. In this state, the side wall (body) of the end mill cuts the curved surface 2 of the nickel cylinder 102 (cutting is performed such that the axis of the nickel cylinder 102 forms right angles with the axis of the end mill). As a result, the edge 3a on the inner-wall-side of the opening 3 is formed acutely and acts as the outer blade. A machining method other than that described above may also be employed, so long as the method enables formation of the opening 3 having the acute edge 3a. For instance, a plurality of nickel cylinders 102 are arranged in a line, and a rotary grindstone is pressed against the nickel cylinders 102 such that the rotating direction of the grindstone becomes perpendicular to the axial direction of each of the nickel cylinders 102, to thus grind portions of the distal ends of the nickel cylinders 102. As a result, the openings 3, each having the acute edge 3a, are formed. The grindstone used for forming the openings 3 preferably has a curved grinding surface to be used for forming the edge 3a.

The outer cylindrical blade 1 is obtained by polishing the inner and outer walls of the nickel cylinder 102.

The opening 12 can also be formed by cutting a portion of the distal end of the metal matrix 100 with the end mill or the like. At this time, the distal end of the end mill is brought into contact with the curved surface 11 of the metal matrix 100, to thus cut the surface. As a result, the edge 12a on the external wall-side of the opening 12 is formed acutely, and acts as an inner blade. Any other method may be employed, so long as the method enables formation of the opening 12 having the acute edge 12a. For instance, it may be a case that the opening 12 is formed before performance of machining involving usage of the end mill, by electric discharge machining, and only finishing of the thus-machined opening is performed through use of the end mill.

The inner cylindrical blade 10 is obtained by polishing the inner and external walls of the metal matrix 100.

In general, when the hollow, cylindrical external wall is machined, precision machining can be performed by cutting or grinding. However, in the case of machining of an inner wall, a highly-versatile machining method or measurement means is not available, and ensuring desired dimensional accuracy is highly difficult. However, in the present embodiment, the shape of the inner wall of the outer cylindrical blade 1 can be formed with high accuracy in conformance with the shape of the external wall of the inner cylindrical blade 10. Consequently, accuracy of meshing engagement between the outer cylindrical blade 1 (outer blade) and the inner cylindrical blade 10 (inner blade) becomes extremely high. Hence, sharp cutting can be performed even at the distal end of the cutter.

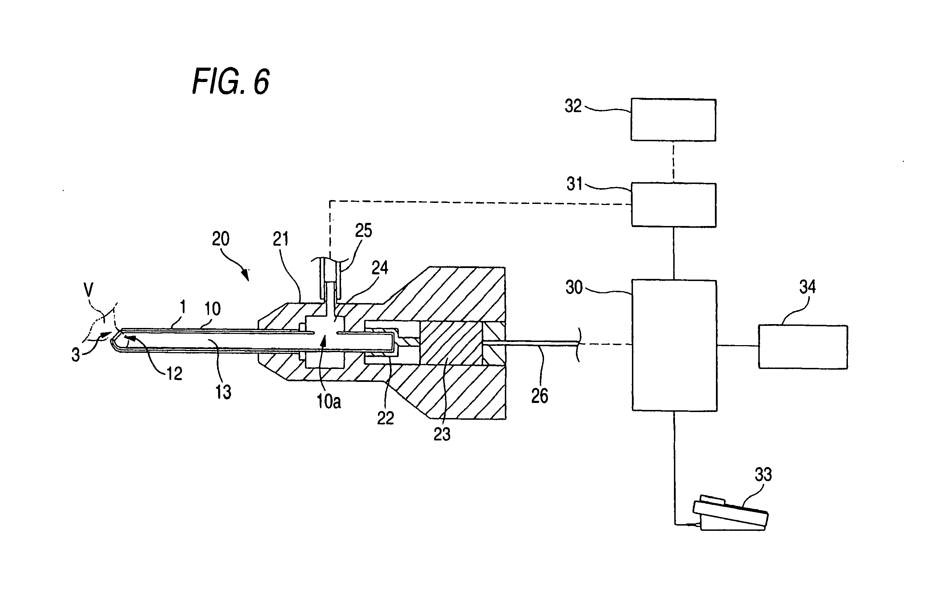

The schematic configuration of the vitreous body cutter using the outer cylindrical blade 1 and the inner cylindrical blade 10 and that of the vitreous body surgical equipment are shown in Fig. 6 and will now be described. The outer cylindrical blade 1 is secured to a housing 21 of the vitreous body cutter 20, which is a handpiece. The inner cylindrical blade 10 is fitted in the outer cylindrical blade 1 so as to be rotatable about the central axis of the inner cylindrical blade. The base end of the inner cylindrical blade 10 has been closed in advance by welding, electrodeposition, or the like. A motor 23 fixed in the housing 21 is connected to the base end via a joint member 22. The motor 23 is electrically connected to a control section 30 of the equipment main body through a cable 26 and rotationally driven in accordance with a control signal from the control section 30. A delivery hole 10a which connects the suction path 13 to a suction chamber 24 provided in the housing 21 is formed in a sidewall of the inner cylindrical blade 10. The suction chamber 24 is connected to a suction pump 31 of the equipment main body through a tube 25, and suction pressure for drawing the vitreous body V by suction develops in the suction path 13 by driving of the suction pump 31.

The equipmentmainbody is roughly divided into the control section 30 for driving and controlling the entire piece of equipment, the suction pump 31 for generating the suction pressure for drawing the vitreous body V by suction, a waste (waste fluid) bag 32, a foot switch 33 for issuing a signal for activating the cutter 20 and the suction pump 31, and a setting panel 34 for setting various surgical conditions. The control section 30 drives and controls the motor 23 and the suction pump 31 on the basis of the signal output from the foot switch 33 or the settings of the setting panel 34.

Operation of the vitreous body surgical equipment having such a configuration will now be described. First, with switches of the setting panel 34, surgical conditions (e.g., a suction pressure, a cutting speed of the cutter 20, and the like) are set. Next, a perfusion liquid from an unillustrated perfusion liquid bottle is introduced into an eye of a patient. Moreover, the outer cylindrical blade 1 of the cutter 20 is inserted into the eye such that the opening 3 is situated at a diseased area, such as an opaque area. Subsequently, the foot switch 33 is stepped on, to thus activate the cutter 20 (the motor 23) and the suction pump 31 at a preset cutting speed and a preset suction pressure.

The motor 2 3 rotates the inner cylindrical blade 10 forward and in reverse while maintaining the preset cutting speed. Forward and rearward rotation of the inner cylindrical blade 10 is for preventing involvement of the vitreous body V. When the inner cylindrical body 10 rotates about its central axis and the opening 3 and the opening 12 overlap each other, the vitreous body V is drawn into the suction path 13 by suction through the opening 3 and the opening 12. The inner cylindrical blade 10 rotates further, whereby the opening 3 and the opening 12 do not overlap each other. As a result, the vitreous body V is incised by meshing engagement between the edge 3a and the edge 12a. The thus-incised vitreous body V is drawn by suction and delivered to the suction chamber 24 by the suction pressured generated by the suction pump 31, through the suction path 13 and the delivery hole 10a. The vitreous body is further discharged to the waste bag 32 through the tube 25.

In the foregoing embodiment, the inner cylindrical blade 10 is arranged to rotate forward and in reverse about its central axis. However, the essential requirement is to be able to bring the opening 3 of the outer cylindrical blade 1 into the open state and the closed state by rotational movement of the inner cylindrical blade 10. For instance, the inner cylindrical blade 10 may be rotated in a single direction about its central axis.

Although in the present embodiment, the inner cylindrical blade 10 is rotated by the motor 23, the inner cylindrical blade 10 may be rotated by providing a mechanism for converting linear motion into rotational motion or through use of air pressure originating from a compressor pump, a solenoid valve, or a diaphragm. Apart of the drive mechanism for rotating the inner cylindrical blade 10 may be provided on the equipment main body.

In the embodiment, in order to efficiently incise the vitreous body located close to a retina, the opening 3 is provided in the distal end of the outer cylindrical blade 1, and the opening 12 is provided in the distal end of the inner cylindrical blade 10. However, needless to say, the present invention can be applied to a vitreous body cutter having an opening in a side surface in the vicinity of the distal end of an outer cylindrical blade, with a view toward enhancing the meshing accuracy between the outer cylindrical blade and the inner cylindrical blade. In this case, the inner cylindrical blade may be axially reciprocated (moved back and forth) with respect to the outer cylindrical blade.

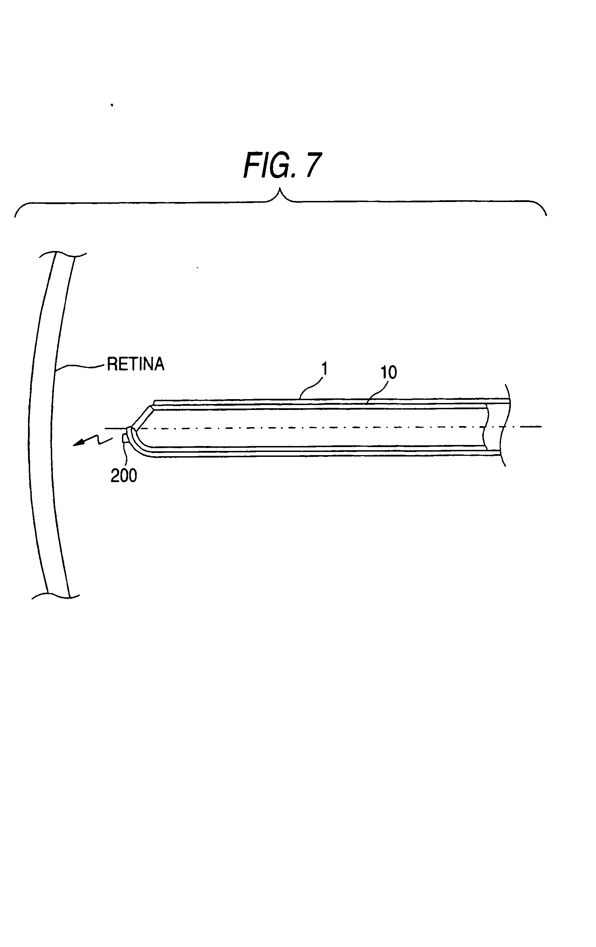

By providing the cutter 20 with a function for detecting and reporting a distance between the distal end of the cutter and the retina, suction of the retina or infliction of damage on the retina, which would otherwise be caused when the cutter 20 approaches too close to the retina, can be prevented. Fig. 7 is a schematic side cross-sectional view showing an embodiment in which a sensor 200 is provided on the vitreous body cutter 20 for detecting a distance between the distal end of the cutter 20 and a retina. The sensor 200 detects a distance by transmitting an ultrasonic signal or an optical signal and receiving a reflected signal. As illustrated, the sensor 200 is disposed on the distal end of the cutter 20 (the outer cylindrical blade 1).

In the cutter 20 having such a configuration, a signal to be received by the sensor 200 is always monitored by the control section 30. When the level of the received signal has reached a predetermined value or more, the control section 30 displays reaching of the predetermined value on a display of the setting panel 34 or issues an alarm sound, thereby informing the user of approach of the cutter 20 to a predetermined distance. Further, the control section 30 may suppress drawing of the retina, by weakening the suction force generated by the suction pump 31 or stop rotation of the inner cylindrical blade 10.

An unillustrated cable for electrically connecting the sensor 200 to the control section 30 can also be embedded in the wall of the outer cylindrical blade 1 (caused to adhere to the wall of the outer cylindrical blade 1) through use of the foregoing electroforming technique. When the cable is embedded in the wall of the outer cylindrical blade 1, electroforming is temporarily suspended when the wall has been formed to a certain thickness during the course of formation of the nickel cylinder 102 shown in Fig. 5B, and the cable sheathed with insulating material is laid on the nickel cylinder 102. Nickel is then deposited to a predetermined thickness by again using electroforming. At this time, the tip end of the cable to be connected to the sensor 200 is preferably covered in advance with a protective member or the like, so that the deposited nickel can be readily removed.

When deposition of nickel on the sheathed cable and embedding the cable in the wall of the outer cylindrical blade 1 are difficult, an insulation portion of the cable to be sheathed may be plated beforehand with nickel or the like. Although the cable is connected to the sensor 200 after the cable and the outer cylindrical blade 1 have been integrated beforehand, it maybe a case that the sensor 200 and the cable, being connected together, are previously provided on the outer cylindrical blade 1, and that only the cable is embedded in the wall of the outer cylindrical blade 1 through use of the foregoing electroforming process.

## Claims

1. A vitreous body cutter (20) for incising a vitreous body in an eye, comprising:
an inner cylindrical blade (10) which has a first
suction hole (12); and
an outer cylindrical blade (1) which has a second suction hole (3), into which the inner cylindrical blade (10) is slidably fitted,
**characterized in that**
the outer cylindrical blade (1) is formed from nickel so as to have a shape of an inner wall formed by transferring a shape of an external wall of the inner cylindrical blade (10) by electroforming.

2. The vitreous body cutter according to claim 1, wherein the first suction hole (12) is formed in a distal end of the inner cylindrical blade (10), and the second suction hole (3) is formed in a distal end of the outer cylindrical blade (1).

3. The vitreous body cutter according to claim 2, wherein the distal ends of the respective cylindrical blades (1, 10) are formed into a shape of a curved surface.

4. The vitreous body cutter according to any one of claims 1 to 3, further comprising:
a drive section (22, 23) for rotating the inner cylindrical blade (10) about its central axis with respect to the outer cylindrical blade (1).

5. Vitreous body surgical equipment comprising:
the vitreous body cutter according to any one of
claims 1 to 4; and
a suction pump (31) for generating a suction pressure within the inner cylindrical blade.

6. A method for manufacturing a vitreous body cutter (20) for incising a vitreous body in an eye, comprising:
a first step of producing a first cylinder (100) which is to be formed into an inner cylindrical blade (10) ;
a second step of producing a second cylinder (102) which is to be formed into an outer cylindrical blade (1); and
a third step of forming openings as suction holes (12, 3) in the produced first (100) and second (102) cylinders to obtain the inner (10) and outer (1) cylindrical blades,
**characterized in that**
the second cylinder (102) is produced so as to have a shape of an inner wall formed by transferring a shape of an external wall of the first cylinder (100) by electroforming in the second step.

7. The manufacturing method according to claim 6, wherein the suction holes (12, 3) are formed in distal ends of the produced first (100) and second (102) cylinders, respectively, in the third step.

8. The manufacturing method according to Claim 6 or 7, wherein the distal ends of the first cylinder (100) is formed into a shape of a curved surface in the first step.

9. The manufacturing method according to any one of claims 6 to 8, wherein the second cylinder (102) is produced by making deposition of nickel on a surface of the first cylinder (100), in the second step.

## Patentansprüche

1. Glaskörper-Schneidegerät (20) zum Einschneiden eines Glaskörpers in einem Auge, umfassend:
eine innere zylindrische Klinge (10), die ein erstes Ansaugloch (12) aufweist; und
eine äußere zylindrische Klinge (1), die ein zweites Ansaugloch (3) aufweist, in welchem die innere zylindrische Klinge (10) gleitfähig eingepasst ist,
**dadurch gekennzeichnet, dass**
die äußere zylindrische Klinge (1) aus Nickel gebildet ist, so dass sie die Form einer Innenwand aufweist, die durch Übertragen einer Form einer Außenwand der innern zylindrischen Klinge (10) durch Galvanoformung ausgebildet wird.

2. Glaskörper-Schneidegerät nach Anspruch 1, wobei das erste Ansaugloch (12) in einem distalen Ende der inneren zylindrischen Klinge (10) ausgebildet ist und das zweite Ansaugloch (3) in einem distalen Ende der äußeren zylindrischen Klinge (1) ausgebildet ist.

3. Glaskörper-Schneidegerät nach Anspruch 2, wobei die distalen Enden der jeweiligen zylindrischen Klingen (1, 10) in einer Form einer gekrümmten Oberfläche ausgebildet sind.

4. Glaskörper-Schneidegerät nach einem der Ansprüche 1 bis 3, ferner umfassend:
einen Antriebsabschnitt (22, 23) zum Drehen der inneren zylindrischen Klinge (10) um ihre Mittelachse in Bezug auf die äußere zylindrische Klinge (1).

5. Glaskörper-Chirurgieinstrument, umfassend:
das Glaskörper-Schneidegerät nach einem der Ansprüche 1 bis 4; und
eine Ansaugpumpe (31) zum Erzeugen eines Ansaugdrucks innerhalb der inneren zylindrischen Klinge.

6. Verfahren zum Herstellen eines Glasköper-Schneidegeräts (20) zum Einschneiden eines Glaskörpers in einem Auge, umfassend:
einen ersten Schritt des Herstellens eines ersten Zylinders (100), der zu einer inneren zylindrischen Klinge (10) geformt werden soll;
einen zweiten Schritt des Herstellens eines zweiten Zylinders (102), der zu einer äußeren zylindrischen Klinge (1) geformt werden soll; und
einen dritten Schritt des Ausbildens von Öffnungen als Ansauglöcher (12, 3) in dem hergestellten ersten (100) und
zweiten (102) Zylinder, um die innere (10) und äußere (1) zylindrische Klinge zu erhalten,
**dadurch gekennzeichnet, dass**
der zweite Zylinder (102) so hergestellt wird, dass er eine Form einer Innenwand aufweist, die im zweiten Schritt durch Übertragen einer Form einer Außenwand des ersten Zylinders (100) durch Galvanoformung ausgebildet wird.

7. Herstellungsverfahren nach Anspruch 6, wobei die Ansauglöcher (12, 3) im dritten Schritt jeweils in distalen Enden des erzeugten ersten (100) und zweiten (102) Zylinders ausgebildet werden.

8. Herstellungsverfahren nach Anspruch 6 oder 7, wobei das distale Ende des ersten Zylinders (100) im ersten Schritt in einer Form einer gekrümmten Oberfläche ausgebildet wird.

9. Herstellungsverfahren nach einem der Ansprüche 6 bis 8, wobei der zweite Zylinder (102) im zweiten Schritt durch Abscheidung von Nickel auf einer Oberfläche des ersten Zylinders (100) hergestellt wird.

## Revendications

1. Moyen de coupe pour corps vitré (20) destiné à inciser un corps vitré dans un oeil, comprenant :
une lame cylindrique intérieure (10) qui comporte un premier trou d'aspiration (12) ; et
une lame cylindrique extérieure (1) qui comporte un second trou d'aspiration (3), dans laquelle la lame cylindrique intérieure (10) est insérée de manière coulissante,
**caractérisé en ce que**
la lame cylindrique extérieure (1) est formée à partir de nickel de manière à avoir une forme d'une paroi intérieure formée en transférant une forme d'une paroi externe de la lame cylindrique intérieure (10) par électroformage.

2. Moyen de coupe pour corps vitré selon la revendication 1, dans lequel le premier trou d'aspiration (12) est formé dans une extrémité distale de la lame cylindrique intérieure (10), et le second trou d'aspiration (3) est formé dans une extrémité distale de la lame cylindrique extérieure (1).

3. Moyen de coupe pour corps vitré selon la revendication 2, dans lequel les extrémités distales des lames cylindriques respectives (1, 10) ont la forme d'une surface incurvée.

4. Moyen de coupe pour corps vitré selon l'une quelconque des revendications 1 à 3, comprenant en outre :
une section d'entraînement (22, 23) destinée à faire tourner la lame cylindrique intérieure (10) autour de son axe central par rapport à la lame cylindrique extérieure (1).

5. Equipement chirurgical pour corps vitré, comprenant :
le moyen de coupe pour corps vitré selon l'une quelconque des revendications 1 à 4 ; et
une pompe d'aspiration (31) destinée à générer une pression d'aspiration dans la lame cylindrique intérieure**.**

6. Procédé de fabrication d'un moyen de coupe pour corps vitré (20) destiné à inciser un corps vitré dans un oeil, comprenant :
une première étape de production d'un premier cylindre (100) qui doit être formé en une lame cylindrique intérieure (10) ;
une deuxième étape de production d'un second cylindre (102) qui doit être formé en une lame cylindrique extérieure (1) ; et
une troisième étape de formation d'ouvertures en tant que trous d'aspiration (12, 3) dans les premier (100) et second (102) cylindres produits afin d'obtenir les lames cylindriques intérieure (10) et extérieure (1),
**caractérisé en ce que**
le second cylindre (102) est produit de manière à avoir une forme d'une paroi intérieure formée en transférant une forme d'une paroi externe du premier cylindre (100) par électroformage à la deuxième étape.

7. Procédé de fabrication selon la revendication 6, dans lequel les trous d'aspiration (12, 3) sont respectivement formés dans les extrémités distales des premier (100) et second (102) cylindres produits, à la troisième étape.

8. Procédé de fabrication selon la revendication 6 ou 7, dans lequel on donne à l'extrémité distale du premier cylindre (100) la forme d'une surface incurvée à la première étape.

9. Procédé de fabrication selon l'une quelconque des revendications 6 à 8, dans lequel le second cylindre (102) est produit en réalisant un dépôt de nickel sur une surface du premier cylindre (100), à la deuxième étape.
